# EUROPEAN PATENT APPLICATION

(11) **EP 4 809 910 A1**
(43) Date of publication of application: **23.09.2026**
(21) Application number: 26164677.2
(22) Date of filing: 13.03.2026
(51) Int. Cl.: A61B 5/0205, A61B 5/08, A61B 5/11, A61B 5/00

(54) **BODY LYING MOVEMENT AND PHYSIOLOGICAL INFORMATION DETECTION APPARATUS**

(30) Priority: 19.03.2025 TW 114110386
(71) Applicant: Decentralized Biotechnology Intelligence Co., Ltd., Taipei 106021 (TW)
(72) Inventor: CHOU, Yao-Sheng, 106021 Taipei City (TW); SU, Wei-Sheng, 106021 Taipei City (TW); LIN, Hsiao-Yi, 106021 Taipei City (TW); JIANG, Lin-Yi, 106021 Taipei City (TW)
(74) Representative: Schmidt, Steffen J.

(57) **Abstract**

The present disclosure provides a body lying movement and physiological information detection apparatus, which includes a piezoelectric pressure sensor array, arranged for sensing the user's vital sign signals and pressure response signals, and determining the user's activity status based on the distribution pattern of the pressure response signals.

## Description

### TECHNICAL FIELD

The present disclosure relates to a physiological information detection apparatus, and more particularly, to a body lying movement and physiological information detection apparatus.

### BACKGROUND

Physiological information refers to information about the human body's condition which includes heart rate, blood pressure, body temperature, respiratory rate, oxygen saturation and other data. Monitoring physiological information helps people understand their physical condition and detect potential health problems in a timely manner so that proper measures can be taken early. Modern technology provides a variety of ways to monitor physiological information, such as smart bracelets, health mobile applications, smart wearable devices, etc., which collect and analyze physiological information through sensors and algorithms. These tools help people monitor physical condition and detect abnormal physiological indicators so as to make corresponding adjustments and treatments.

With the advancement of technology and the change of human living habits, the field of home care has received more and more attention. The physical condition of the care recipient can be monitored at any time. These physiological data may be uploaded to the hospital's monitoring system via the Internet, thereby providing more complete health care services for the home care recipient, so that family members or doctors may understand the physical condition of the care recipient (for example: infants, those who are unable to take care of themselves, those with limited mobility or the elderly) at any time. In the event of an emergency, the sensor will also actively send a signal to family members and the emergency rescue center.

Taking SIDS (sudden infant death syndrome) as an example, the above emergency situations are sudden and unexpected deaths of healthy infants (defined by the American Academy of Pediatrics AAP as infants under 1 year old). Regardless of the medical history, physical examination, scene investigation and autopsy, the cause of death cannot be explained or discovered, and it is classified as SIDS. An epidemiological survey conducted by the National Institute of Child Health and Human Development (NICHD) in the United States shows that the incidence rate is approximately 0.5 to 1.2 per thousand. The average age of onset is 11 weeks old; most cases occur between 2 and 4 months, and 90% occur before 6 months of age. It happens all over the world, regardless of race or region. The incidence rate is higher in male infants, and the disease usually occurs in the middle of the night or early morning, mostly while the infant is sleeping, and is common in autumn, winter and early spring.

According to statistics, the high-risk groups for SIDS include: (1) mothers under 20 years old, unmarried, poor, no prenatal examinations, no adequate medical care during pregnancy, abnormal weight increasing before delivery, short interval between two pregnancies, sick during pregnancy, miscarriage history, smoked or took drugs, etc.; (2) the risk of premature babies is 3 to 4 times that of full-term babies; (3) newborns are too low in birth weight; (4) sleeping on their stomachs; (5) sleeping in soft beds or in overheated environments; (6) male babies are more likely to die than female babies; (7) babies sleep in the same bed with adults or other children; and (8) family history of sudden infant death syndrome.

In order to prevent the care recipient (for example, infants, those who are unable to take care of themselves, limited mobility or the elderly) from experiencing emergency medical situations, the priority is to stably monitor vital signs, such as heart rate, breathing, and issue an alarm notification when an abnormal situation occurs; secondly, it is possible to determine the activity status of the care recipient, such as sitting, lying, prone, turning over, leaving, etc. However, current products related to the medical care for the care recipient, such as mattresses, fail to take into account comprehensive monitoring of vital signs and activity status.

Therefore, what is required is to provide physiological information detection device for home care to monitor the breathing, heart rate and other vital signs of the care recipient, and to determine the activity status of the care recipient at any time.

### SUMMARY

In order to improve the above deficiencies, according to one aspect of the present disclosure, the disclosure discloses a body lying movement and physiological information detection apparatus, including a pressure sensor array, which is configured to sense a user's vital signal, a pressure response signal or the combination thereof, and to determine the user's activity state (or a posture state) by the distribution pattern of the pressure response signal. The body lying movement and physiological information detection device includes a mattress or a belly band. In some examples, the activity states can be determined through one or more analysis processes, such as obtaining a multi-region pressure response by the pressure sensor array, generating distribution pattern of the multi-region pressure response, performing a posture classification, and sending an alarm notification when an abnormal condition is detected.

In one embodiment, the pressure sensor array includes piezoelectric, capacitive, resistive sensors or any combination thereof. The body lying movement and physiological information detection apparatusincludes a system circuit board electrically connected to the piezoelectric pressure sensor array and connected to an external mobile device for transmitting the vital sign signals, the pressure response signals or the combination thereof. The vital sign signals include the user's breathing signal, heart rate signal. In some examples, the activity state (or posture state) includes at least one of the following: sitting, lying, prone, standing up, turning over, or a combination thereof. Generally, the activity state in the present disclosure may broadly refer to activities related to home care scenarios or the postures exhibited by the user.

In one embodiment, the aforementioned body lying movement and physiological information detection apparatusincludes a flexible substrate, having at least a first sensing area and a second sensing area arranged on a first side of the flexible substrate, wherein a pressure sensor array is distributed in the first sensing area and the second sensing area; the flexible substrate covers the user's body so that the pressure sensor array contacts the user's chest, back or both.

In one embodiment, the flexible substrate includes a third sensing area, wherein the third sensing area contacts the abdomen of the user. The activity state of the user is determined by the distribution pattern of the response signals from the first sensing area, the second sensing area, the third sensing area or any combination thereof. The first sensing region includes a piezoelectric pressure sensor array, the second and the third sensing regions include the capacitive or resistive pressure sensor array.

### BRIEF DESCRIPTION OF THE DRAWINGS

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion.
FIG. 1 shows a body lying movement and physiological information detection device according to one embodiment of the present disclosure.
FIG. 2 shows a body lying movement and physiological information detection device according to an alternative embodiment of the present disclosure.
FIG. 3 is a schematic diagram showing the pressure response distribution measured by the present disclosure when the user is lying down (left) and sitting (right).
FIG. 4A-FIG. 4C are schematic diagrams showing the distribution of pressure responses measured by the present disclosure when the user is lying down, sleeping on their stomachs and sitting.
FIG. 5 shows the arrangement of the pressure sensor array and circuit board of the body lying motion and physiological information detection device (mattress) of the present disclosure.
FIG. 6A shows the arrangement of the pressure sensor array and circuit board of the bellyband of the present disclosure.
FIG. 6B shows the arrangement of the pressure sensor array and circuit board of the bellyband of the present disclosure.
FIG. 7 shows an example of the design of a separate heart sound sensor for the body lying movement and physiological information detection device of the present disclosure.
FIG. 8 shows a functional block diagram of the body lying movement and physiological information detection device of the present disclosure.
FIG. 9 shows an example of a processing system of the present disclosure.

### DETAILED DESCRIPTION

Some preferred embodiments of the present disclosure will now be described in greater detail. However, it should be recognized that the preferred embodiments of the present disclosure are provided for illustration rather than limiting the present disclosure. In addition, the present disclosure can be practiced in a wide range of other embodiments besides those explicitly described, and the scope of the present disclosure is not expressly limited except as specified in the accompanying claims.

The present disclosure discloses the body lying movement and physiological information detection apparatus, which is used to monitor the user's breathing, heart rate, and other vital sign signals in real time and to determine the user's activity status. Given that current mattress products do not take into account the monitoring of vital signs and most activity states, for example, they are unable to determine activity states such as lying down and sitting. The present disclosure discloses a mattress and a bellyband, which can be used alone or together to monitor the user's breathing, heart rate and other vital signs and to determine the user's activity status at any time.

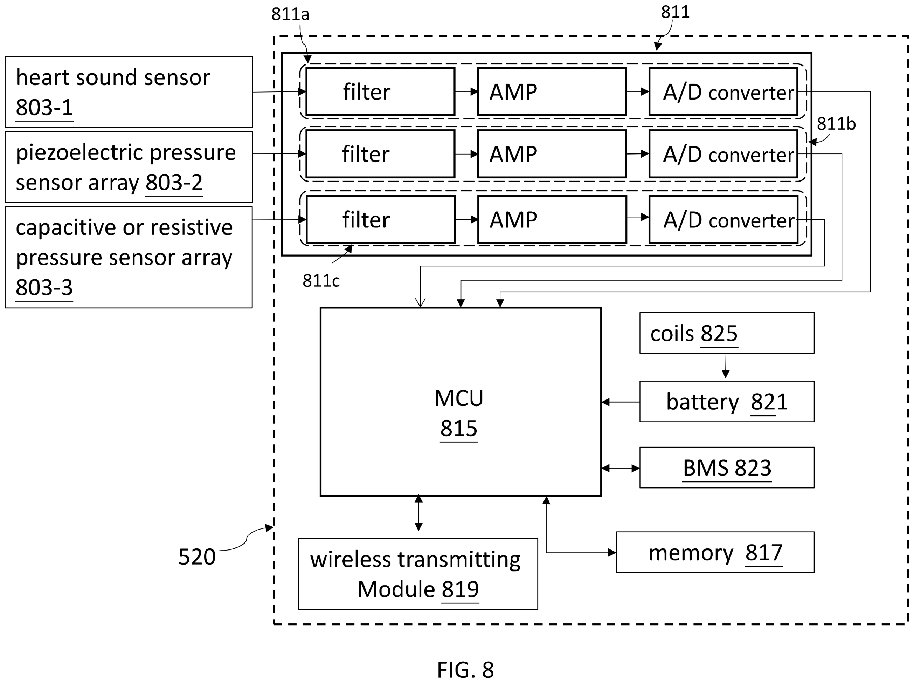

Please refer to FIG. 8, which shows a functional block diagram detection device of the present disclosure, it includes a heart sound sensor 803-1 for detecting the heart sound signal of the user, such as the care recipient. The pressure sensor array can be selected from one or any combination of piezoelectric pressure sensor array, capacitive pressure sensor array, or resistive pressure sensor array. In one embodiment, the present disclosure includes a piezoelectric pressure sensor array 803-2 and/or a capacitive or resistive pressure sensor array 803-3. For example, the piezoelectric pressure sensor array 803-2 detects ballistocardiogram (BCG) signals and the pressure distribution data; the capacitive or resistive pressure sensor array 803-3 detects the pressure distribution data. The above-mentioned heart sound signals, BCG signals, and pressure distribution data are filtered, amplified, and (analog/digital) converted individually through the corresponding signal preprocessing channels (preprocess channels) 811a, 811b, 811c in the signal preprocessing circuit 811 to obtain preprocessed digitized heart sound signals, BCG signals, and pressure distribution data.

In one embodiment, each pre-processing channel 811a, 811b, 811c includes a filter, a signal amplifier, and an analog/digital (A/D) converter. The microprocessor 815 stores the stable and background noise-free cardiac sound signals, BCG signals, and pressure distribution data in the storage unit 817 through instructions or programs, or send the signals to an external device for further analysis via the wireless transmission module 819. The external devices can be smartphones, tablets, cloud servers, etc.

The battery pack 821 provides power for the body lying movement and physiological information detection apparatus, and cooperates with the power management module 823 to optimize the use of power. The battery pack 821 is also wirelessly charged by the charging coil 825.

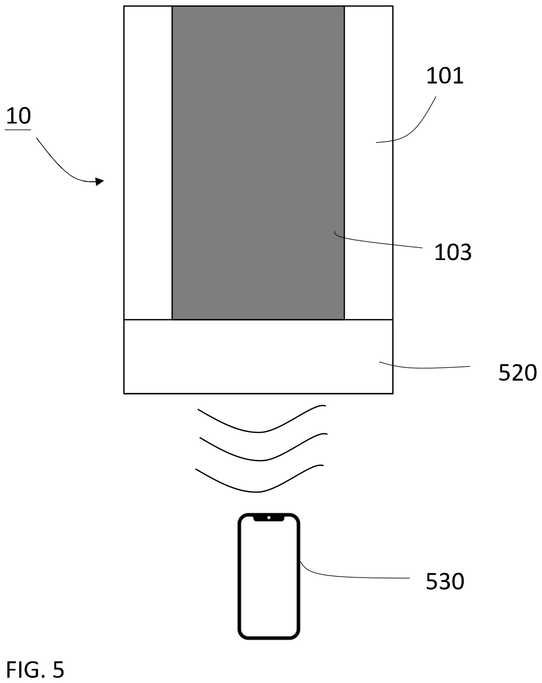

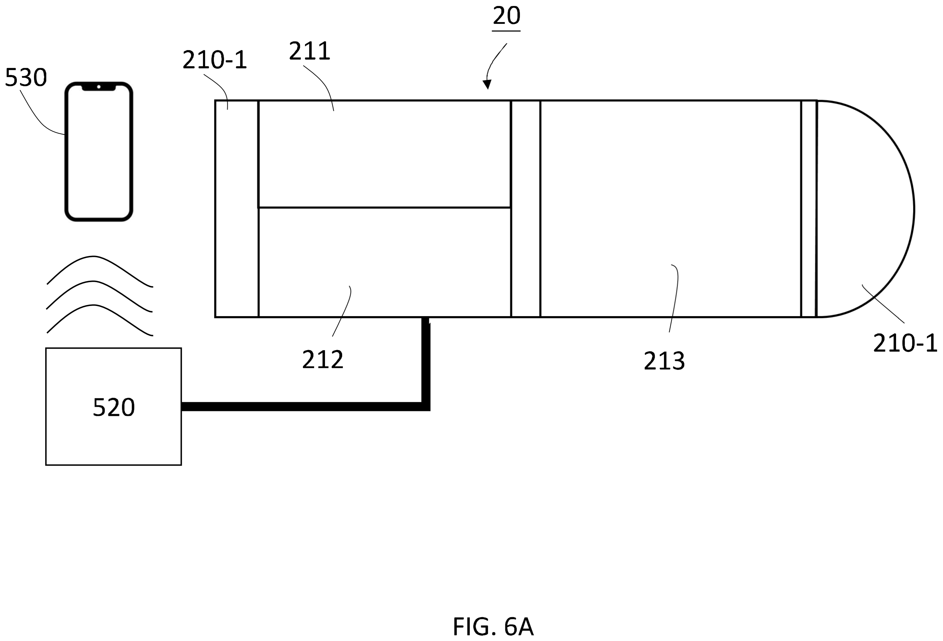

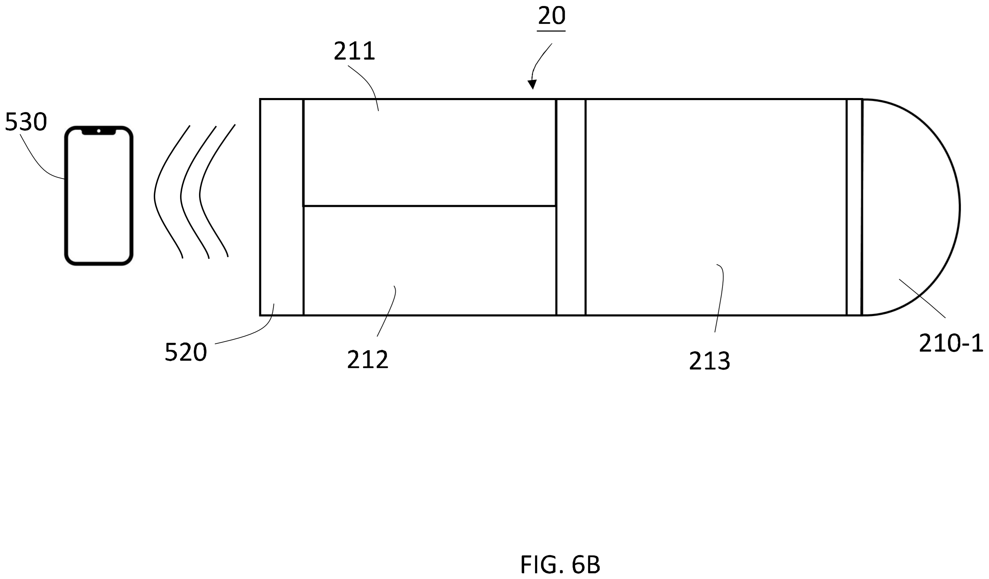

In one embodiment, the aforementioned microprocessor 815, the storage unit 817, the wireless transmission module 819, the signal preprocessing circuit 811, and the power management module 821 are integrated into a single system circuit board, such as the circuit board 520 shown in FIGS. 5, 6A and 6B.

In one embodiment, the user may view the collected BCG signals, PCG signals, and pressure distribution data through the external computing device, and further analyze and compare the data by the external computing device. The external computing device may be a smart phone, a tablet computer, or a cloud server. The body lying movement and physiological information detection apparatus can be applied to the mattress 10 or the belly band 20.

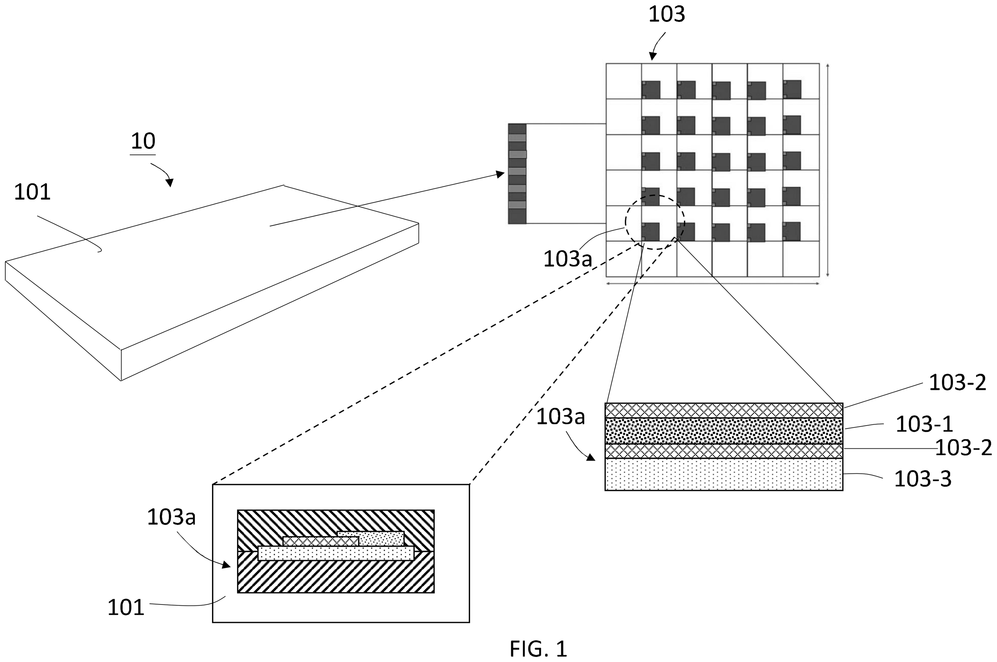

Referring to FIG. 1, one embodiment of the present disclosure is a mattress 10, which includes a mattress body 101 and a pressure sensor array 103, wherein the pressure sensor array 103 is disposed on the surface of the mattress body 101 or embedded in the mattress body 101. The pressure sensor array 103 is composed of a plurality of pressure sensors 103a, wherein the pressure sensors 103a include piezoelectric sensors, capacitive sensors, or resistive sensors to detect the pressure. Referring to FIG. 1, the pressure sensor 103a, taking a piezoelectric sensor as an example, includes a piezoelectric material 103-1, such as polyvinylidene fluoride (PVDF) polymer piezoelectric film, lead zirconate titanate (PZT), and other materials. The upper and lower surfaces thereof are plated with conductive metals (e.g., aluminum (Al), copper (Cu), etc.) as electrodes 103-2, formed on a flexible substrate 103-3, for sensing voltage signals generated by vibration. The pressure sensor 103a is not limited to the piezoelectric sensor. Referring to FIG. 1, the pressure sensor 103a may also be composed of a thin film type (including a capacitor or a resistor) and packaged inside the mattress body 101.

In one embodiment, the pressure sensor array 103 composed of piezoelectric sensors is taken as an example, and is configured to monitor vital sign signals such as breathing and heart rate. Because the piezoelectric frequency response of the piezoelectric sensor is fast and sensitive, it can be used to capture the user's BCG signal and extract the BCG time difference. The detected BCG signal can be separated into a breathing signal and a heart rate signal through signal processing. In addition, due to the configuration of the pressure sensor array 103, the user's activities, such as sitting, lying, prone, standing, turning over or the combination thereof, can be sensed and determined using the capacitance changes of the piezoelectric sensor electrodes. That is, the user's activity status can be determined based on the distribution pattern of the pressure response signal detected by the pressure sensor array 103. Similarly, for resistive type sensors, the user's sitting, lying, prone, standing, turning over, or the combination thereof can also be determined by detecting the distribution pattern of the pressure response signal obtained by the change in resistance. According to an embodiment of the present disclosure, the pressure sensor array 103 includes piezoelectric sensors, capacitive sensors, or resistive sensors, which can be manufactured by a bonding packaging process.

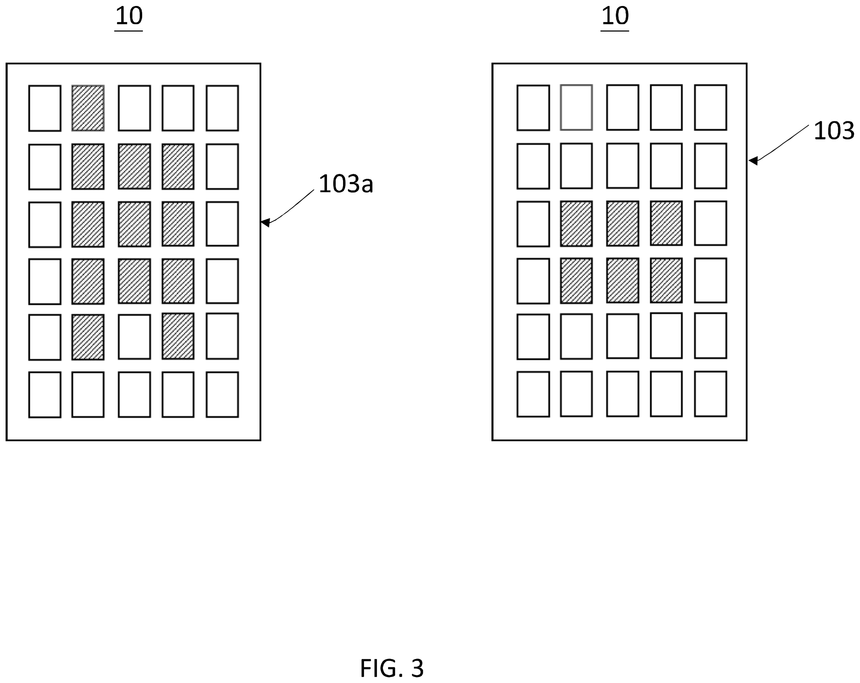

Regarding the application scenario of the mattress 10 as the body lying movement and physiological information detection apparatus, refer to FIG. 3, it shows the pressure response distribution measured by the pressure sensor array 103 (refer to Figure 1) when the user is lying down (left) and sitting (right). In addition, it should be emphasized that no matter what activity state the user is in, the piezoelectric pressure sensor array 103 disposed on the mattress 10 will sense the user's BCG signals and heart sound signals.

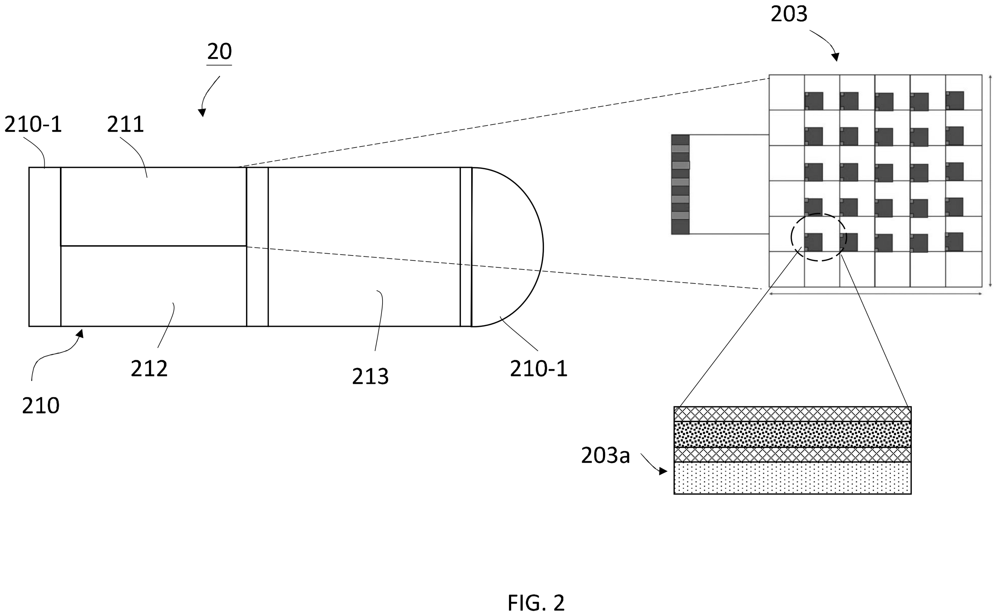

Referring to FIG. 2, another embodiment of the present disclosure is a belly band 20, which mainly comprises a sheet (e.g., fabric or flexible cushion) 210 for being disposed adjacent to at least a portion of the user's body. For instance, the sheet 210 may cover the user's body (e.g., chest and/or abdomen). In some examples, a multi-region pressure data can be obtained for generating distribution features and performing a posture classification. For instance, a first sensing area 211, a second sensing area 212, and a third sensing area 213 are defined on one surface of the sheet 210, corresponding respectively to the user's chest, abdomen and/or back when the sheet covers the user's body. A Velcro or fastener is provided at both ends 210-1 of the sheet 210 for fixing the belly band; a pressure sensor array 203 composed of piezoelectric sensors is provided on the first sensing area 211, and pressure sensor arrays 203 composed of capacitive or resistive sensors are respectively provided on the second sensing area 212 and the third sensing area 213 (i.e., a first pressure sensor array and a second pressure sensor array). The pressure sensor array 203 composed of piezoelectric sensors is used to detect the user's BCG signals and heart sound signals. It is used to monitor the user's vital signs, and also detect pressure response signals (i.e., first pressure response signals). The first pressure response signal is combined with the second and third pressure response signals detected by the pressure sensor arrays arranged in the second sensing area 212 and the third sensing area 213. According to their distribution patterns (or pressure response distributions), a posture classification operation can be performed, and therefore the distribution patterns can be used to determine the user's activities such as sitting, lying, prone, leaving, turning over, or the combination thereof. In some examples, these users' activities may reflect situations such as the user getting out of bed, standing up, falling, experiencing apnea, prolonged immobility, sleeping on one's side or back, being in a semi-recumbent position, and other similar situations. The principle is the same as that described in FIG. 1, therefore, redundancy description is omitted.

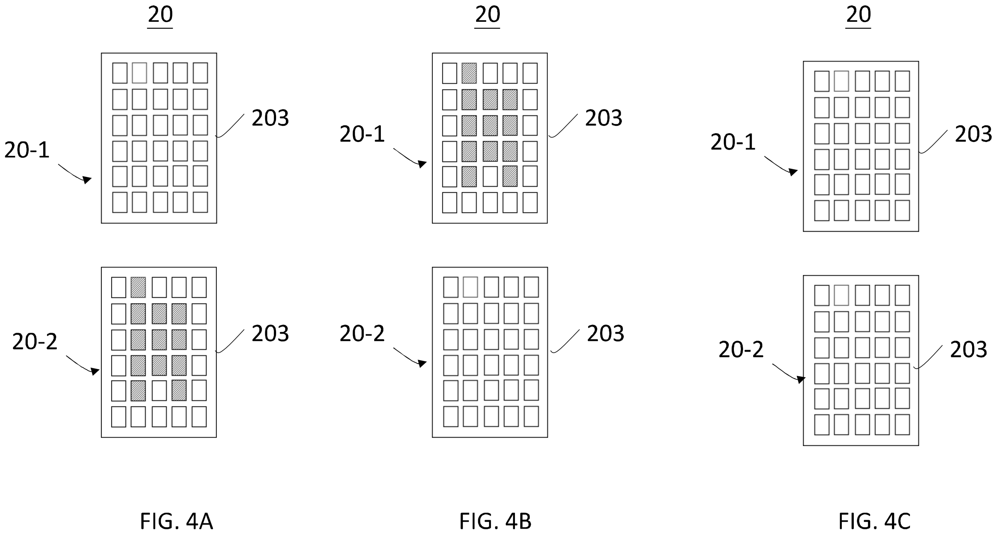

In the belly band 20 embodiment, refer to FIGS. 4A to 4C, which respectively show schematic diagrams of pressure response distribution measured when the user is lying down, sitting and sleeping on their stomachs. FIG. 4A shows that when the user is lying down, the pressure sensor array 203 disposed on the back side 20-2 of the belly band 20 detects the pressure generated by the user's weight and it generates a corresponding pressure response distribution, while the pressure sensor array 203 disposed on the front side 20-1 of the belly band 20 is not subjected to force and therefore, no pressure is detected. FIG. 4B shows that when the user sleeps on his/her stomach, the pressure sensor array 203 disposed on the front side 20-1 of the belly band 20 will sense the pressure generated by the user's weight and the corresponding pressure response distribution is generated, while the pressure sensor array 203 disposed on the back side 20-2 of the belly band 20 will not detect any pressure. FIG. 4C shows that when the user is sitting, the pressure sensor arrays 203 disposed on the front side 20-1 of the belly band 20 and the back side 20-2 of the belly band 20 do not measure any pressure. Similarly, it should be emphasized that no matter what activity state the user is in, the piezoelectric pressure sensor array 203 disposed on the chest of the front side 20-1 of the belly band 20 will sense the user's BCG signals and heart sound signals.

FIG. 5 shows a configuration diagram of the pressure sensor array 103 and the circuit board 520 of the mattress 10 according to one embodiment of the present disclosure, wherein the circuit board 520 is electrically connected to the pressure sensor array 103 and integrated into the mattress body. The circuit board 520 of the mattress 10 communicates with the external device 530 through the wireless transmission module 891 (such as WiFi, Bluetooth, etc. 5G, 6G standard), and the external device includes, for example, a smart phone, a tablet computer, a cloud server, and other external computing electronic devices. The vital signs data (including heart sound signals and BCG signals) and pressure response distribution data collected by the body lying movement and physiological information detection apparatus, such as the mattress 10, can be transmitted to the external device 530 for further processing and analysis via the wireless transmission module 891 (see FIG. 8). According to an embodiment of the present disclosure, BCG signals can be processed by the BCG signal processing module to obtain heart rate signals which is separated from the breathing signals. When the vital sign data or the activity state is abnormal, the present disclosure, such as the mattress 10, sends an alarm notification via an alarm element connected to the circuit board 520 signal (i.e., triggering alarms notification under abnormal conditions).

Referring to FIG. 8, the wireless transmission module 891 includes a short-range wireless transmission module, such as WiFi, Bluetooth, etc. In another embodiment, the wireless transmission module 891 includes a long-distance wireless transmission module that can communicate directly with the cloud or remote server. The long-distance wireless transmission module includes a 4G, 5G or 6G communication protocol. In this embodiment, the wireless transmission module 891 may require a physical SIM or an eSIM.

Figure 6A shows the belly band 20 embodiment of the present disclosure, it shows a configuration diagram of a pressure sensor array 203 and a circuit board 520, wherein the circuit board 520 is electrically connected to the pressure sensor array 203 of the first sensing area 211, the second sensing area 212 and the third sensing area 213 arranged on the belly band 20 (refer to Figure 2), the circuit board 520 and the belly band 20 are configured separately. The circuit board 520 is electrically connected to the pressure sensor array 203 via a lead. Similarly, the circuit board 520 of the belly band 20 communicates with the external device via the wireless transmission module. The vital signs data (including heart sound signals and BCG signals) and pressure response distribution data collected by the belly band 20 can be transmitted to the external device 530 via the wireless transmission module 891 for further processing and analysis.

Figure 6B shows a configuration diagram of the pressure sensor array 203 and the circuit board 520 of the belly band 20 of the present disclosure, wherein the circuit board 520 is electrically connected to the pressure sensor array 203 arranged in the first sensing area 211, the second sensing area 212 and the third sensing area 213 of the belly band (refer to Figure 2), and the circuit board 520 and the belly band 520 are integrated into a whole. In addition, the circuit board 520 of the belly band 20 is wirelessly connected to the external device 530 through the wireless transmission module 891. The external device 530 includes external computing electronic devices such as smart phones, tablet computers, and cloud servers. In another embodiment, the present disclosure does not need to communicate with the cloud server through a mobile device. The belly band 20 can directly communicate with the external device 530 by a built-in long-distance wireless transmission module. The long-distance wireless transmission module includes 4G, 5G or 6G communication protocols. In this embodiment, the belly band 20 may require a physical SIM or an eSIM. The vital signs data collected by the belly band 20 includes the heart sound signals and BCG signals, as well as pressure response distribution data, which are transmitted to the external device 530 via short-distance or long-distance wireless transmission for further processing and analysis.

According to an embodiment of the present disclosure, the BCG signals can be processed by the BCG signal processing module to obtain heart rate signals and separate respiratory signals. When the vital sign data is abnormal, the belly band 20 sends out the alarm notification via the alarm element connected to the circuit board 520.

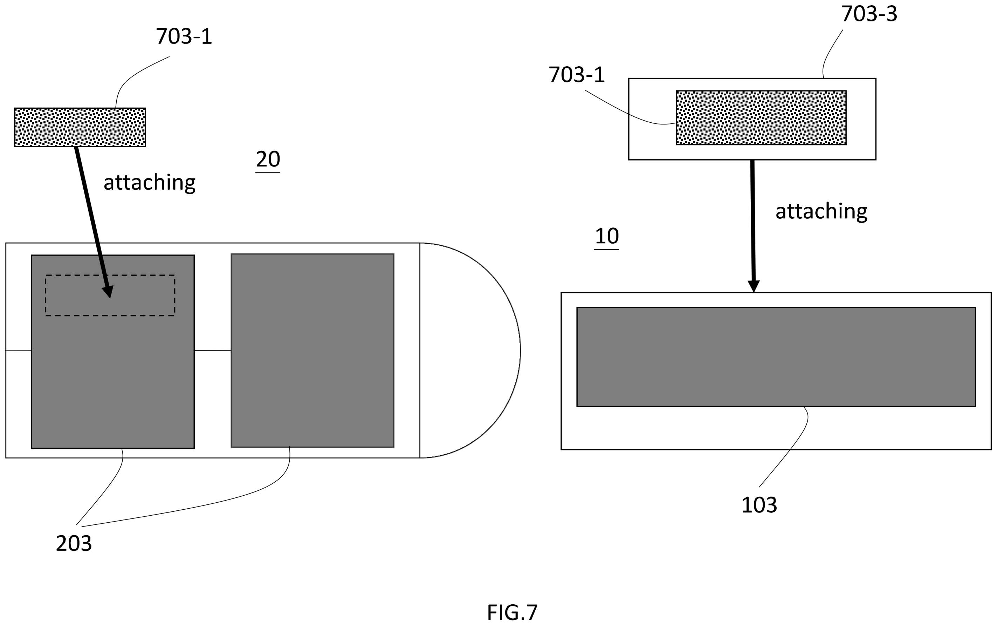

FIG. 7 shows an example of the design of a separate heart sound sensor 703-1 for the mattress 10 and the belly band 20 of the present disclosure; the left figure shows that the heart sound sensor 703-1 can be set on the chest position of the belly band 20 by attaching (refer to FIG. 2); the right figure shows that the heart sound sensor 703-1 is formed on one surface of the flexible substrate 703-3, and the other surface of the flexible substrate 703-3 can be attached to the mattress 10 and the belly band 20 by Velcro or a patch. According to an embodiment of the present disclosure, the heart sound sensor 703-1 may be the piezoelectric sensor.

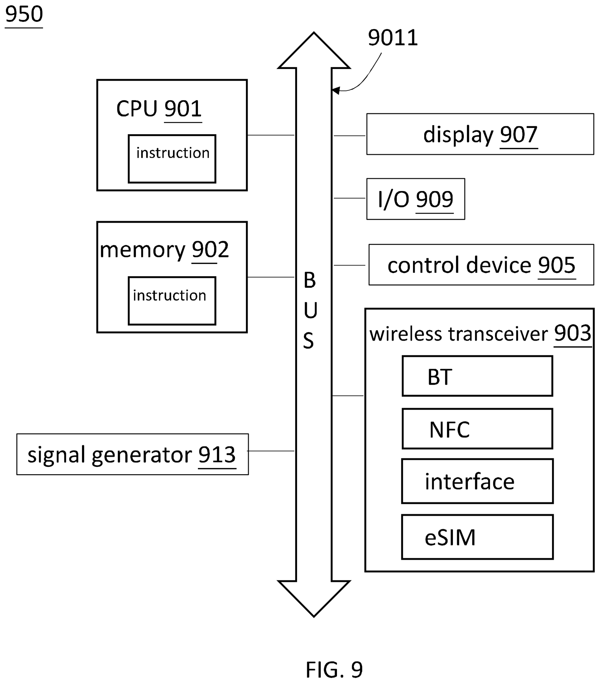

FIG. 9 shows the functional block diagram of a processing system 950 of an external device 530. Specifically, the processing system 950 refers to a computing system in the external device 530, the processing system 950 executes instructions to perform calculations according to the embodiment of the present disclosure, such as executing the previously described algorithm for analyzing BCG signals, extracting the characteristics of cardiac sound signals (PCG), and displaying pressure response distribution data. Those skilled in the art should understand that the above instructions may be stored and/or executed as hardware, software or firmware without departing from the spirit of the present disclosure. Furthermore, those skilled in the art should appreciate that the exact configuration of each processing system may be different, and the processing system 950 shown in FIG. 9 is only an example.

The processing system 950 includes a processor 901, a main memory 902, a wireless transceiver 903 (including a Bluetooth module, a near field communication (NFC) module, etc., and a wireless network interface), a control device 905 (such as a keyboard and a pointing device), a video display 907, an input/output (I/O) device 909, and a signal generating device 913 that is communicatively connected to a Bus 9011.

The main memory 902, the wireless transceiver 903, the video display 907, the input/output (I/O) device 909, and any number of other peripheral devices are connected to the processor 901 and exchange data with the processor 901 through the Bus 9011 for use in the application program executed by the processor.

The wireless transceiver 903 is connected to the antenna for sending and receiving voice, data signals via a wireless telecommunication channel. In one embodiment, the wireless telecommunication channel may be a digital wireless telecommunication channel, such as WiFi, Bluetooth, RFID, NFC, 4G/5G/6G or any other future wireless communication interface.

The video display 907 receives display data from the processor 901 and displays images on the screen for the user to watch. The video display 907 may be a liquid crystal display (LCD) or an organic light emitting diode (OLED) display.

The main memory 902 is a device that sends and receives data to and from the processor 901 and stores the data. The main memory 902 may include non-volatile memory, such as read-only memory (ROM), which stores instructions and data needed to operate the individual subsystems of the processing system and to boot the system at startup. Those skilled in the art will appreciate that any number of memories may be used to perform this function. The main memory 902 may also include volatile memory, such as random-access memory (RAM), which stores instructions and data required by processor 901 to execute software instructions for computing processes. It should be note that any type memory could be used as volatile memory, and the type used for an application is a design choice to the skilled person.

The Bluetooth module allows the processing system 950 to establish communication with the device based on the Bluetooth technology standard. Other peripheral devices that may be connected to processor 601 include Global Positioning System (GPS) and other positioning transceivers.

A processor 901 refer to CPU, a microprocessor, or any combination thereof, which executes processing operation instructions according to the present disclosure. The processor 901 is capable of executing various application programs stored in the storage unit. These applications can receive user input via a display having a touch screen or directly from a keyboard area. Some of the applications stored in the main memory 902 and executable by the processor 901 may be applications developed by UNIX, Android, iOS, Windows, Blackberry, or other platforms.

One of the benefits of the present disclosure is to provide the dual-function sensing (physiological signals, gestures) for many application scenarios The present disclosure simultaneously detects physiological signals (such as heart sounds, BCG, breathing) and changes in body posture (such as sitting, lying, turning over, sleeping on one's stomach, etc.). It is particularly suitable for the scenarios, for example, newborn care, elder fall monitoring, and postoperative patient bed monitoring. The unique practical value of the present disclosure cannot be achieved by any single-function prior arts. Additionally, the present disclosure may integrate functional multiplayers including the sensing layer (pressure sensor), the signal layer (BCG/PCG), the analysis layer (posture judgment), the transmission layer (WiFi/4G/5G/eSIM) and the application layer (home care alarm) in one architecture, thereby forming a closed-loop system from data acquisition to abnormal alarm. It is not a simple combination of a single module, and the unexpected results cannot be achieved by any existing prior arts.

While various embodiments of the present disclosure have been described above, it should be understood that they have been presented by a way of example and not limitation. Numerous modifications and variations within the scope of the disclosure are possible. The present disclosure should only be defined in accordance with the following claims and their equivalents.

## Claims

1. A body lying movement and physiological information detection apparatus, **characterized by** comprising:
a flexible substrate (210) having a first sensing area and a second sensing area formed on a first side of the flexible substrate (210);
a pressure sensor array (203) distributed across the first sensing area (211) and the second sensing area (212), wherein the flexible substrate (210) is configured to be disposed adjacent to at least a portion of a user's body; and
wherein the pressure sensor array (203) is configured to sense a vital sign signal, a pressure response signal, or a combination thereof in order to obtain a vital sign of the user, an activity state of the user, or a combination thereof, and
wherein the body lying movement and physiological information detection apparatus is configured for use with a mattress (10) or a belly band (20).

2. The body lying movement and physiological information detection apparatus of claim 1, wherein the pressure sensor array (203) includes piezoelectric sensors, capacitive sensors, resistive sensors, or a combination thereof.

3. The body lying movement and physiological information detection apparatus of claim 1, further comprising a system circuit board (520) electrically connected to the pressure sensor array (203).

4. The body lying movement and physiological information detection apparatus of claim 3, wherein the system circuit board (520) is electrically connected to an external device to transmit the vital sign signal, the pressure response signal, or the combination thereof.

5. The body lying movement and physiological information detection apparatus of claim 1, wherein the vital sign signal includes a breathing signal.

6. The body lying movement and physiological information detection apparatus of claim 1, wherein the vital sign signal includes a heart rate signal.

7. The body lying movement and physiological information detection apparatus of claim 1, wherein the activity state comprises sitting, lying, prone, standing, turning over, or a combination thereof, and the activity state is obtained by at least one of the following operations:
obtaining a multi-region pressure response by the pressure sensor array;
generating distribution pattern of the multi-region pressure response;
performing a posture classification; and
sending an alarm notification when an abnormal condition is detected.

8. The body lying movement and physiological information detection apparatus of claim 1, wherein the flexible substrate (210) further has a third sensing area (213).

9. The body lying movement and physiological information detection apparatus of claim 8, wherein the third sensing area (213) contacts the user's abdomen.

10. The body lying movement and physiological information detection apparatus of claim 9, wherein an activity state of the user is determined based on a response signal of the first sensing area (211), the second sensing area (212), the third sensing area (213), or a combination thereof.

11. The body lying movement and physiological information detection apparatus of claim 10, wherein the first sensing area (211) includes a piezoelectric pressure sensor array.

12. The body lying movement and physiological information detection apparatus of claim 11, wherein the second sensing area (212) includes a capacitive pressure sensor array or a resistive pressure sensor array.

13. The body lying movement and physiological information detection apparatus of claim 12, wherein the third sensing area (213) includes the capacitive pressure sensor array or the resistive pressure sensor array.

14. The body lying movement and physiological information detection apparatus of claim 1, further comprising a wireless communication module (903) selected from the group consisting of WiFi, blue tooth, 4G, 5G, and 6G protocols.

15. The body lying movement and physiological information detection apparatus of claim 14, further comprising a physical SIM or an eSIM.
